(19) Europäisches Patentamt
European Patent Office
Office européen des brevets

(11) Veröffentlichungsnummer: **0 091 402**
**B1**

(12) # EUROPÄISCHE PATENTSCHRIFT

(45) Veröffentlichungstag der Patentschrift: **26.02.86**

(21) Anmeldenummer: **83810127.7**

(22) Anmeldetag: **28.03.83**

(51) Int. Cl.⁴: **C 07 D 491/147**, B 41 M 5/16, B 41 M 5/18, B 41 M 5/26, C 07 D 491/052 // (C07D491/052, 311:00, 231:00), (C07D491/052, 311:00, 235:00)

(54) **Chromenoazaidolizine, Verfahren zu ihrer Herstellung und ihre Verwendung in druck- oder wärmeempfindlichen Aufzeichnungsmaterialien.**

(30) Priorität: **06.04.82 CH 2124/82**

(43) Veröffentlichungstag der Anmeldung: **12.10.83 Patentblatt 83/41**

(45) Bekanntmachung des Hinweises auf die Patenterteilung: **26.02.86 Patentblatt 86/9**

(84) Benannte Vertragsstaaten: **BE CH DE FR GB IT LI**

(56) Entgegenhaltungen: **EP-A-0 033 716** **FR-A-2 381 768**

(73) Patentinhaber: **CIBA-GEIGY AG, Klybeckstrasse 141, CH-4002 Basel (CH)**

(72) Erfinder: **Balli, Heinz, Prof. Dr., Höhenstrasse 2, CH-4125 Riehen (CH)**
Erfinder: **Gunzenhauser, Sigmund, Dr., Ziegelackerweg 3, CH-4144 Arlesheim (CH)**
Erfinder: **Fletcher, Ian John, Dr., Bürgenstal 27, CH-4312 Magden (CH)**
Erfinder: **Bedekovic, Davor, Dr., Stefanstrasse 12, CH-4106 Therwil (CH)**

**EP 0 091 402 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft Chromenoazaindolizin-Verbindungen, Verfahren zu ihrer Herstellung und ihre Verwendung als Farbbildner in druckempfindlichen oder wärmeempfindlichen Aufzeichnungsmaterialien.

Die Chromenoazaindolizin-Verbindungen entsprechen den allgemeinen Formeln

(1a) 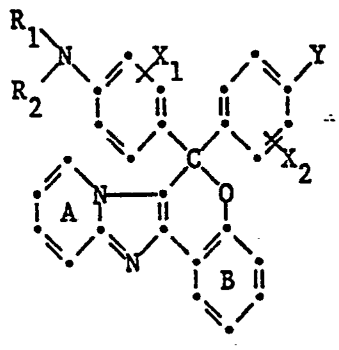 oder 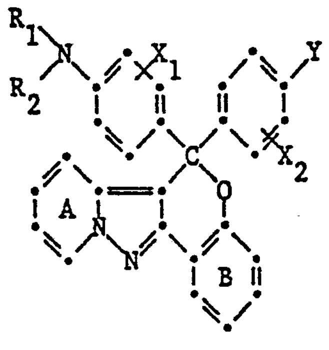 (1b)

worin

Y, $X_1$ und $X_2$, unabhängig voneinander, je Wasserstoff, Halogen, Niederalkyl, Niederalkanoylamino oder die Gruppen der Formeln

(1c) $-OR_3$ oder 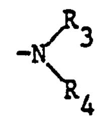 (1d),

$R_1$,$R_2$,$R_3$ und $R_4$ unabhängig voneinander Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl, Benzyl oder durch Halogen, Nitro, Niederalkyl oder Niederalkoxy substituiertes Phenyl oder Benzyl oder die Substituentenpaare ($R_1$ und $R_2$) und $R_3$ und $R_4$), unabhängig voneinander, je zusammen mit dem sie verbindenden Stickstoffatom einen gesättigten, fünf- oder sechsgliedrigen heterocyclischen Rest, bedeuten und

die Ringe A und B unabhängig voneinander unsubstituiert oder durch Halogen, Nitro, Niederalkyl, Niederalkoxy, Phenyl, Phenoxy oder durch eine gegebenenfalls durch Niederalkyl, Phenyl, Niederalkanoyl oder Benzyl mono- oder disubstituierte Aminogruppe substituiert sind, wobei

die Bezeichnung Nieder $C_1$-$C_5$ darstellt.

Vorzugsweise ist Y eine Gruppe der Formel (1d). Die Gruppen der Formeln $-NR_1R_2$ und $-NR_3R_4$ können voneinander verschieden sein oder sind vorzugsweise identisch. $X_1$ und $X_2$ sind ebenfalls vorzugsweise gleich.

Niederalkyl und Niederalkoxy stellen bei der Definition der Reste der Chromenoazaindolizine solche Gruppen oder Gruppenbestandteile dar, die 1 bis 5, insbesondere 1 bis 3 Kohlenstoffatome aufweisen, wie z.B. Methyl, Ethyl, n-Propyl, Isopropyl, n-Butyl, sek.-Butyl oder Amyl bzw. Methoxy, Ethoxy oder Isopropoxy.

Niederalkanoylamino weist 2 bis 5 Kohlenstoffatome auf, wie z.B. Acetylamino, Propionylamino oder Butyrylamino. Halogen in Verbindung mit sämtlichen vor- und nachstehenden Substituenten bedeutet beispielsweise Fluor, Brom oder vorzugsweise Chlor.

Stellen die Substituenten $R_1$, $R_2$, $R_3$ und $R_4$ Alkylgruppen dar,so können sie geradkettige oder verzweigte Alkylreste sein. Beispiele für solche Alkylreste sind Methyl, Ethyl, n-Propyl, Isopropyl, n-Butyl, sek.-Butyl, Amyl, n-Hexyl, 2-Ethyl-hexyl, n-Octyl, Isooctyl oder n-Dodecyl.

Sind die Alkylreste in $R_1$, $R_2$, $R_3$ und $R_4$ substituiert, so handelt es sich vor allem um Cyanoalkyl, Halogenalkyl, Hydroxyalkyl oder Alkoxyalkyl jewels vorzugweise mit insgesamt 2 bis 4 Kohlenstoffatomen, wie z.B. ß-Cyanoethyl, ß-Chloroethyl, ß-Hydroxyethyl, ß-Methoxyethyl oder ß-Ethoxyethyl.

Beispiele für Cycloalkyl in der Bedeutung der R-Reste sind Cyclopentyl oder vorzugsweise Cyclohexyl.

Bevorzugte Substituenten in der Benzylgruppe und in der Phenylgruppe der R-Reste sind z.B. Halogen, Nitro, Methyl oder Methoxy. Beispiele für derartige araliphatische bzw. aromatische Reste sind p-Methylbenzyl, o-oder p-Chlorbenzyl, o- oder p-Nitrobenzyl, o- oder p-Tolyl, Xylyl, o-, m- oder p-Chlorphenyl, o- oder p-Nitrophenyl oder o- oder p-Methoxyphenyl.

Wenn die Substituentenpaare ($R_1$ und $R_2$) und ($R_3$ und $R_4$) jeweils zusammen mit dem gemeinsamen Stickstoffatom einen heterocyclischen Rest darstellen, so ist dieser beispielsweise Pyrrolidino, Piperidino,

Pipecolino, Morpholino, Thiomorpholino oder Piperazino.

Die Substituenten $R_1$, $R_2$, $R_3$ und $R_4$ sind vorzugsweise Benzyl,Niederalkyl oder Cyano-niederalkyl, z.B. ß-Cyanoethyl.

$X_1$ und $X_2$ bedeuten vorzugsweise jeweils Wasserstoff. Sie können jedoch vorteilhafterweise auch Methyl, Methoxy, Ethoxy, Chlor oder Acetylamino sein, die sich vorzugsweise in ortho-Stellung zur Kohlenstoffbindung befinden.

Der stickstoffhaltige Ring A ist vorzugsweise nicht weiter substituiert.

Der Benzolring B ist vorzugsweise unsubstituiert oder ein- oder zweifach durch Halogen, Niederalkyl oder Niederalkoxy, z.B. durch Chlor, Methyl, Isopropyl, tert.-Butyl oder Methoxy substituiert. Diese Substituenten befinden sich vorzugsweise in para-Stellung bzw. in orthound para-Stellung zum Sauerstoff.

Praktisch wichtige Chromenoazaindolizin-Verbindungen entsprechen der Formel

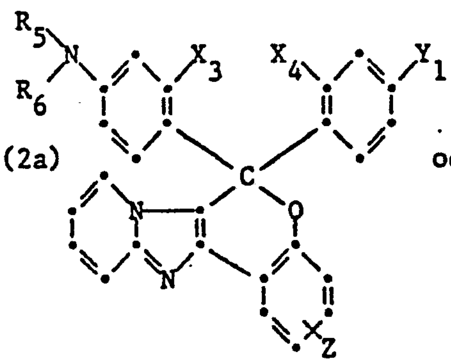

(2a)

oder

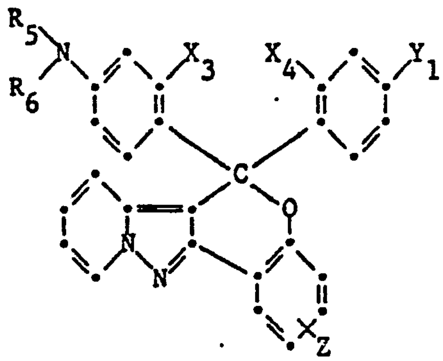

(2b)

worin

$Y_1$ Wasserstoff, $-OR_7$ oder

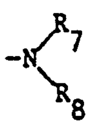

.

$X_3$ und $X_4$ unabhängig voneinander Wasserstoff, Halogen, Methyl, Methoxy, Ethoxy oder Acetylamino,

$R_5$,$R_6$,$R_7$

und $R_8$ unabhängig voneinander Niederalkyl, Cyanoethyl, Benzyl oder Phenyl

oder

die Substituentenpaare ($R_5$ und $R_6$) und ($R_7$ und $R_8$) unabhängigvoneinander Pyrrolidino, Piperidino oder Morpholino

und Z Wasserstoff, Halogen, Niederalkyl, Niederalkoxy, Phenyl oder eine gegebenenfalls durch Niederalkyl oder Benzyl oder Phenyl mono- oder disubstituierte Aminogruppe

bedeuten.

Unter diesen Verbindungen der Formeln (2a) und (2b) sind diejenigen besonders bevorzugt, bei denen $Y_1$ die Gruppe der Formel $-NR_7R_8$ darstellt,

$X_3$ und $X_4$ Wasserstoff sind und

Z Wasserstoff, Halogen, Methyl oder Methoxy bedeutet.

Von ganz besonderem Interesse sind Chromenoazaindolizine der Formeln

(3a) 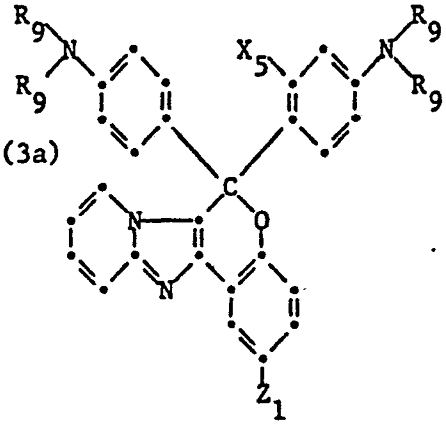 oder 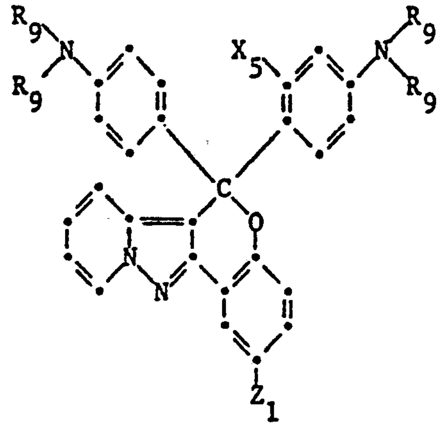 (3b)

worin

$R_9$ Niederalkyl, insbesondere Methyl oder Ethyl, $X_5$ Wasserstoff, Methoxy, oder Ethoxy und $Z_1$ Wasserstoff, Methyl, Methoxy oder Halogen bedeuten.

Unter diesen Verbindungen der Formeln (3a) und (3b) sind diejenigen besonders bevorzugt, bei denen $R_9$ Methyl oder Ethyl, $X_5$ Wasserstoff und $Z_1$ Wasserstoff, Chlor, Methyl oder Methoxy bedeuten.

Die erfindungsgemässen Chromenoazaindolizine der Formel (1a) können dadurch hergestellt werden, dass man eine 1-Azaindolizinver-bindung der Formel

(4a) 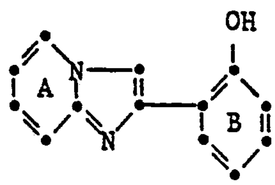

oder eine 1-Azaindoliziniumverbindung der Formel

(4b) 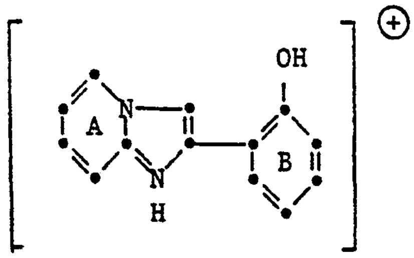 An $^{\ominus}$

mit einer Carbinolverbindung der Formel

(5) 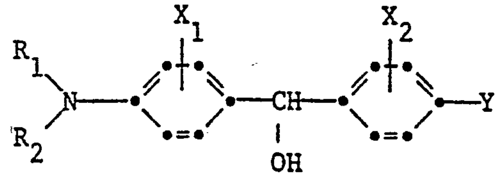

umsetzt und enschliessend oxidiert, worin A, B, $R_1$, $R_2$, $X_1$, $X_2$ und Y die angegebene Bedeutung haben und An $\ominus$ das Anion einer organischen oder vorzugsweise anorganischen Säure, z.B. das Chlorid-, Bromid-, Fluorid-, Sulfat-, Phosphat- oder insbesondere Perchloration bedeutet.

Die Reaktion wird vorzugsweise so durchgeführt, dass man zuerst die Reaktionskomponenten in einem organischen Lösungsmittel, das mit Wasser mischbar und inert gegenüber den Ausgangsstoffen ist, zur Reaktion bringt und dann nach Zugabe einer wässerigen Lösung eines Alkalimetallcarbonates oder Alkalimetallhydroxids

das Umsetzungsprodukt mit einem Oxidationsmittel zur Chromenoverbindung oxidiert.

Bei Verwendung der 1-Azaindolizinverbindung der Formel (4a) verläuft die Umsetzung vorzugsweise in Anwesenheit eines sauren Katalysators z.B. einer niederen aliphatischen Carbonsäure wie Ameisensäure oder Essigsäure oder einer anorganischen Säure wie Salzsäure, Schwefelsäure, Phosphorsäure oder Perchlorsäure.

Die Umsetzung kann bei einer Temperatur von 10° bis 100° C, vorzugsweise 20 bis 40° C vorgenommen werden. Die Oxidationsstufe kann bei Tempereturen von 0° bis 100°C erfolgen. Vorteilhafterweise oxidiert man im Bereich zwischen Raumtemperatur (20 bis 25° C) und 100° C, vorzugsweise bei Raumtemperatur.

Als organische Lösungsmittel eignen sich zweckmässigerweise niedrige aliphatische Alkohole, wie z.B. Methanol, Ethanol oder Isopropanol oder cyclische Ether,wie z.B. Dioxan oder Tetrahydrofuran, γ-Butyro-lacton Acetonitril Dimethylsulfoxid, Dimethylformamid, Dimethylacetamid oder insbesondere N-Methylpyrrolidon.

Als Alkalimetallcarbonate kommen z.B. Kaliumcarbonat oder Natriumcarbonet, als Alkelimetallhydroxid in erster Linie Kaliumhydroxid oder Natriumhydroxid in Betracht.

Als Oxidationsmittel kommen beispielsweise Chromate, Bichromate, Chlorate, Chlorite, Peroxide, Mangandioxid, Bleidioxid, Chlor, Brom, molekularer Sauerstoff, Luft, Perborete, Permanganate, Wasserstoffperoxid, Chloranil und insbesondere wasserlösliche Salze des Hexacyanoferrat-III in Frage. Als wasserlösliche Salze des Hexacyanoferrat-III eignen sich vor allem die des Kaliums und Natriums. Anstelle des handelsüblichen Trikalium-hexacyanoferrat-III können auch Lösungen verwendet werden, die durch Oxidation von Kaliumhexacyanoferrat-II in Gegenwart von Säuren mit Wasserstoffperoxid bei Raumtemperetur erhalten werden.

Die Menge an wasserlöslichem Salz des Hexecyanoferret-III beträgt mindestens die stöchiometrisch erforderliche Menge. Vorzugsweise verwendet man das 1,0 bis 1,2-feche der stöchiometrisch erforderlichen Menge, d.h. 2,0 bis 2,4 Mol Hexacyanoferrat-III je Mol der Reaktionskomponenten.

Nach Beendigung der Oxidation erfolgt die Isolierung der Chromenoazaindolizin-Verbindung, indem man das rohe Produkt aus der Reaktionsmischung durch Abfiltrieren entfernt und mit Wasser neutral wäscht. Die erhaltene Chromeno-1-azeindolizin-Verbindung wird dann über Kieselgelplatten oder dünnschichtchromatogrephisch und/oder durch Umkristallisation gereinigt.

Die Chromeno-3-azaindolizine der Formel (1b) werden dadurch hergestellt, dass man eine 3-Azaindolizinverbindung der Formel

(6) 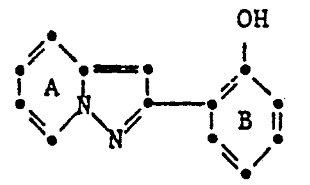 ,

worin A und B die angegebene Bedeutung haben, mit einer Carbinolverbindung der Formel (5) umsetzt und anschliessend oxidiert.

Diese Umsetzung erfolgt ebenfalls vorteilhafterweise in einem polaren organischen Lösungsmittel, besonders in niederen Alkanolen, in cyclischen Ethern wie z.B. Dioxan, Tetrahydrofuran, Dimethylsulfoxid, Acetonitril, Dimethylformamid, γ-Butyrolacton oder N-Methylpyrrolidon und vorzugsweise in Anwesenheit eines sauren Katalysators. Die Umsetzung kann bei Raumtemperatur (20° bis 25° C) vorgenommen werden. Als saure Katalysatoren eignen sich z.B. niedere aliphatische Carbonsäuren wie Ameisensäure oder Essigsäure und anorganische Säuren wie Salzsäure, Schwefelsäure, Phosphorsäure oder Perchlorsäure sowie auch Phosphoroxichlorid.

Das Umsetzungsprodukt kann, falls erwünscht, isoliert werden.

Die Oxidation des Umsetzungsproduktes zu der Chromeno-3-azaindolizin-Verbindung der Formel (Ib) erfolgt mit Oxidationsmitteln. Geeignete Oxidationsmittel sind auch hier z.B. Chromate, Bichromate, Chlorate, Chlorite, Peroxide, Mangandioxid, Bleidioxid, molekularer Sauerstoff, Luft, Perborate, Permanganate, Wasserstoffperoxid und Chloranil.

Die besten Ergebnisse in bezug auf Ausbeute und Reinheit der erhaltenen Chromeno-3-azaindolizine erzielt man mit einem wasserlöslichen Salz des Hexacyanoferrat-III und vor allem mit Trikaliumhexacyanoferrat-III, das vorzugsweise nach Zugabe einer wässerigen Lösung eines Alkalimetallcarbonates oder vor allem eines Alkalimetallhydroxides, z.B. Kaliumhydroxid, und zweckmässigerweise in Gegenwart von N-Methylpyrrolidon angewandt wird.

Die Oxidationstemperatur richtet sich in der Regel nach dem Oxidationsmittel und nach dem Siedepunkt des eventuell verwendeten Lösungsmittels. Sie liegt zweckmässig zwischen 20 und 100° C. Bei Verwendung von Kaliumhexacyanoferrat-III verläuft die Oxidation vorzugsweise bei Raumtemperatur.

Die 1-Azaindolizine der Formel (4a) können gemäss L. Schmid und K. Gründig, Monatshefte der Chemie, Band 84, Seiten 491-497 hergestellt werden. Ein bevorzugtes Verfahren zur Herstellung der 1-Azaindolizine der Formel (4a) besteht darin, dass man ein 2-Halogenacetylphenol der Formel

(7) 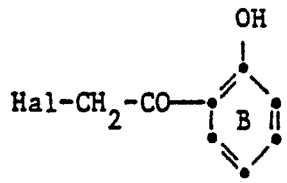

worin B die angegebene Bedeutung hat und Halogen z.B. Brom, Jod oder Chlor bedeutet, mit einer 2-Aminopyridinverbindung der Formel

(8) 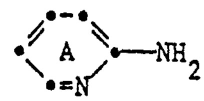

worin A die angegebene Bedeutung hat, umsetzt. Darauf kann die erhaltene 1-Azaindolizinverbindung in an sich bekannter Weise in das erwünschte Cycloammoniumsalz der Formel (4b) überführt werden.

Bevorzugte Ausgangsstoffe der Formel (4a) und (4b) sind 2-(2-Hydroxy-phenyl)-1-aza-indolizine bzw.-1-azaindoliziniumverbindungen, deren Hydroxyphenylreste unsubstituiert oder durch Methoxy, tert.-Butyl oder insbesondere durch Chlor oder Methyl ringsubstituiert sind.

Die als Ausgangsstoffe eingesetzten 3-Azaindolizine der Formel (6) können dadurch hergestellt werden, dass man beispielsweise eine 2-(Alkoxy-phenacyl)-pyridinverbindung der Formel

(9) 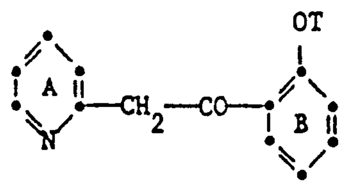 ,

worin T $C_1$-$C_4$-Alkyl, insbesondere Methyl, bedeutet, mit einer nach Y. Tamura, J. Minamikawa, K. Sumoto, S. Fujii, M. Ikeda, J. Org.Chem. 38, 6, 1973, 1239-41 hergestellten Lösung von Mesitylsulfonylhydroxylamin in Dichlormethan zur 2-(Alkoxy-phenyl)-3-azaindolizin-Verbindung der Formel

(10) 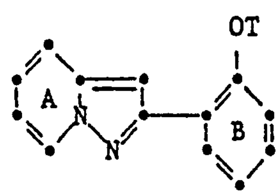

cyclisiert und diese mit Trimethylsilyljodid bei einer Temperatur von 30 bis 120° C zu der Verbindung der Formel (6) entalkyliert.

Die 2-(Alkoxyphenyl)-3-azaindolizinverbindungen der Formel (10) können auch hergestellt werden, indem man gemäss der DE-OS 2 118 917 die entsprechende Schiff'sche Base eines 1-Amino-2-picoliniumsalzes oder gemäss Bower, J.Chem.Soc. 1957, 4510 eine 2-(β-Amino-2'-alkoxy-phenylethyl)-pyridinverbindung zur 3-Azaindolizinverbindung der Formel (10) oxidiert.

Bevorzugter Ausgangsstoff der Formel (5) ist 4,4'-Bis-(dimethylamino)-benzhydrol ("Michler's Hydrol").

Die Chromenoazaindolizine der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie intensive blaue, grünblaue oder grüne Farbtöne, die ausgezeichnet sublimationsund lichtecht sind. Sie sind deshalb auch sehr wertvoll im Gemisch mit einem oder mehreren anderen bekannten Farbbildnern, z.B. 3,3-(Bis-aminophenyl-)phthaliden, 3,3-(Bis-indolyl-)phthaliden, 3-Aminofluoranen, 2,6-Diaminofluoranen, Leukoauramine, Spiropyranen, Spirodipyranen, Chromenoindolen, Phenoxazinen, Phenothiazinen, Carbazolylmethanen oder weiteren Triarylmethan-leukofarbstoffen, um blaue, marine-blaue, graue oder schwarze Färbungen zu ergeben.

Die Chromenoazaindolizine der Formeln (1) bis (3) zeigen sowohl auf phenolischen Unterlagen, wie auch besonders auf aktivierten Tonen, eine verbesserte Farbintensität und Lichtechtheit. Sie eignen sich vor allem als

sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit,Siliciumdioxyd, Aluminiumoxid, Aluminiumsulfat, Aluminiumphosphat, Zinkchlorid, aktiviertes Kaolin oder irgendein beliebiger Ton. Bevorzugte Entwickler sind sauer reagierende, organische Verbindungen, wie z.B. gegebenenfalls ringsubstituierte Phenole, Salicylsäure oder Salicylsäureester und deren Metallsalze, ferner ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxypolymethylen. Es können auch Mischungen der genannten polymeren Verbindungen eingesetzt werden. Besonders bevorzugte Entwickler sind Zinksalicylateoder die Kondensationsprodukte von p-substituierten Phenolen mit Formaldehyd. Die letzteren können auch Zink enthalten.

Die Entwickler können zusätzlich auch mit anderen, an sich unreaktiven oder wenig reaktiven Pigmenten gemischt eingesetzt werden. Beispiele für solche Pigmente sind: Talk, Titandioxid, Zinkoxid, Kreide, Tone wie Kaolin, sowie organische Pigmente, z.B. Harnstoff-Formaldehyd oder Melamin-Formaldehyd-Kondensationsprodukte.

Der Farbbildner liefert an den Punkten, an denen er mit dem Elektronenakzeptor in Kontakt kommt, eine gefärbte Markierung. Um zu verhindern, dass die Farbbildner, die in dem druckempfindlichen Aufzeichnungsmaterial enthalten sind, frühzeitig aktiv werden, werden sie in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes, zerbrochen werden und wenn die Farbbildnerlösung auf diese Weise auf ein benachbartes Blatt übertragen wird, das mit einem Elektronenakzeptor beschichtet ist, wird eine farbige Stelle erzeugt. Diese Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek.-Butyl oder tert.-Butyl alkylierte Derivate von Diphenyl, Naphthalin oder Triphenyl, Dibenzyltoluol, Terphenyl, partiell hydriertes Terphenyl, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. aus Gelatine und Gummi arabicum bestehen kann, wie dies z.B. in der US-Patentschrift 2 800 457 beschrieben ist. Die Kapseln können vorzugsweise auch aus einem Aminoplast oder modifizierten Aminoplasten durch Polykondensation gebildet werden, wie es in den britischen Patentschriften 959,264, 1 156 725, 1 301 052 und 1 355 124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke oder Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind,

verwendet.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten wie Elektrocardiographen, verwendet. Die Bilderzeugung (Markierung) kann auch manuell mit einer erhitzten Feder erfolgen. Eineweitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bereichenmittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und besonders Phenolharze, oder auch phenolische Verbindungen, wie sie beispielsweise in der DE-PS 12 51 348 beschrieben sind, z.B. 4-tert.-Butylphenol, 4-Phenylphenol, 4-Hydroxydiphenyläther, α-Naphthol, β-Naphthol, 4-Hydroxybenzoesäuremethylester, 4-Hydroxyacetophenon, 2,2'-Dihydro-xydiphenyl, 4,4'-Isopropylidendiphenol, 4,4'-Isopropyliden-bis-(2-methylphenol), 4,4'-Bis-(hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z.B. Weinsäure, Oxalsäure, Maleinsäure, Zitronensäure, Citraconsäure und Bernsteinsäure.

Vorzugsweise werden zur Herstellung des thermoreaktiven Aufzeichnungsmaterials schmelzbare, filmbildende Bindemittel verwendet, Diese Bindemittel sind normalerweise wasserlöslich, während die Chromenoazaindolizine und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyäthylcellulose, Methylcellulose, Carboxymethylcellulose, Polyacrylamid, Polyvinylpyrrolidon, Gelatine und Stärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff:Formaldehydpolymerisate, enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Paraffinwachs, Polyethylenwachs.

In den folgenden Beispielen beziehen sich die angegebenen Prozentsätze, wenn nichts anderes angegeben ist, auf das Gewicht.

**Beispiel 1**

**6,6-Bis(4-dimethylamino-phenyl)-2-methyl-6H-chromeno [4,3-b]-1-azaindolizin**

650 mg 1H-2-(2-Hydroxy-5-methylphenyl)-1-azaindoliziniumperchlorat und 540 mg 4,4'- Bis-(dimethylamino)-benzhydrol werden in 15 ml 1-Methyl-pyrrolidon-(2) bei Raumtemperatur 3 1/2 Stunden gerührt und mit 6 ml wässriger 1N Kaliumhydroxidlösung versetzt. Als dann werden während 30 Minuten 1,32 g Trikaliumhexacyanoferrat in 20 ml Wasser zutropfen gelassen. Das Rohprodukt wird durch langsame Zugabe von 60 ml Wasser unter Rühren gefällt und abfiltriert. Nach der Reinigung über Kieselgel-Platten mit Toluol/Ethylacetat (3:1) und anschliessender Umkristallisation aus Methanol unter Zusatz von etwas Ammoniak, viel Wasser und etwas Kaliumchlorid erhält man 380 mg (40% der Theorie) einer Verbindung der Formel

(11) 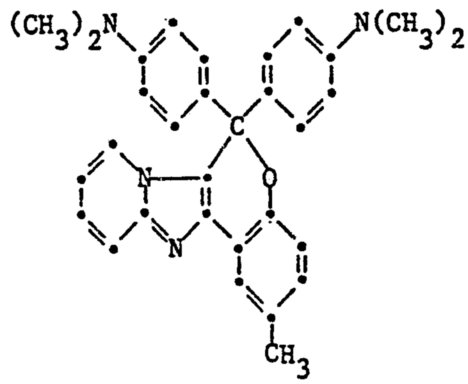

mit einem Schmelzpunkt von 241-243°C. Auf Säureton entwickelt dieser Farbbildner eine intensive blaue Farbe.

Das in Beispiel 1 veiwendete 1-Azaindoliziniumperchloratsalz wird folgendermassen hergestellt:

Je 10 mMol 2-Bromacetyl-4-methylphenol und 2-Aminopyridin werden 2-3 Stunden auf 100°C erhitzt. Das erhaltene Rohprodukt wird zur Entfernung von braunen Nebenprodukten mit wenig Aceton digeriert, mit Ether versetzt und abfiltriert. Der Rückstand wird in Ethanol aufgenommen, mit 70%i-ger Perchlorsäure und bis zur Trübung mit Wasser versetzt. Das Produkt scheidet sich in hellbeigen Nadeln ab. Die Ausbeute beträgt 2,21 g (68% der Theorie). Die erhaltene Verbindung der Formel

(i) 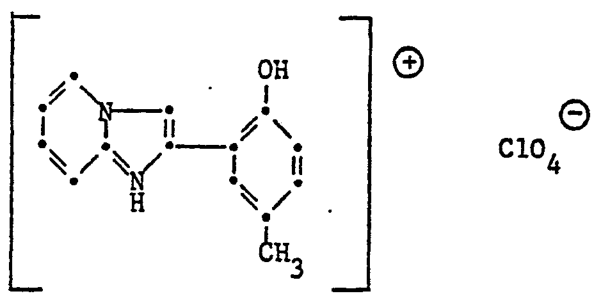

hat einen Schmelzpunkt von 231-235°C.

**Beispiel 2**

**6,6-Bis(4-dimethylaminophenyl)-6H-chromeno[4,3-b]-1-azaindolizin**

Ersetzt man im Beispiel 1 das 1-Azaindoliziniumperchlorat der Formel (i) durch 2,8 g des Bromides der Formel

(ii) 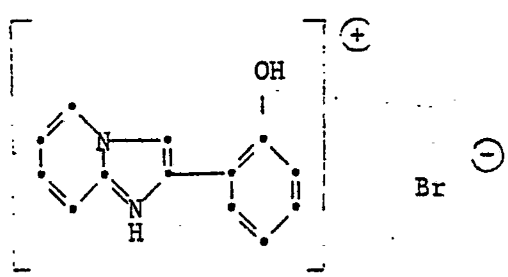 Smp. 253-255°C

und verfährt im übrigen wie in Beispiel 1 beschrieben, so erhält man 2,5 g der Verbindung der Formel

(12)

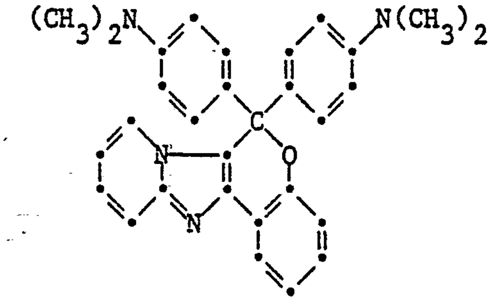

mit einem Schmelzpunk von 268 - 271° C. Auf Säureton entwickelt dieser Farbbildner eine intensive blaue Farbe.

**Beispiel 3**

**6,6-Bis(4-dimethylaminophenyl)-2-methyl-6H-chromeno[3,4-a]-3azaindolizin**

450 mg 2-(2-Hydroxy-5-methyl-phenyl)-3-azaindolizin und 540 mg 4,4'-Bis-(dimethylamino)-benzhydrol werden in 15 ml 1-Methylpyrrolidon-(2) gelöst, mit 280 mg 70%iger Perchlorsäure versetzt und bei Raumtemperatur 3 Stunden gerührt. Hierauf werden 6 ml wässerige IN Kaliumhydroxidlösung zugegeben und während 30 Minuten eine Lösung von Trikaliumhexacyanoferrat in 20 ml Wasser zutropfen gelassen. Anschliessend wird das Reaktionsgemisch tropfenweise mit 60 ml Wasser verdünnt, worauf der Niederschlag abfiltriert und in Toluol aufgelöst wird. Die Toluollösung wird über wasserfreiem Kaliumcarbonat getrocknet und über 100 g Kieselgel chromatographiert, indem zuerst mit Toluol einige Nebenprodukte eluiert werden. Das Hauptprodukt wird mit Toluol/Ethylacetat (25:1) eluiert.

Nach dem Eindampfen bleiben 390 mg (41% der Theorie) farblose, dünnschicht-chromatographisch reine Kristalle zurück. Umkristallisiert aus Cyclohexan/Hexan hat die Verbindung der Formel

(13)

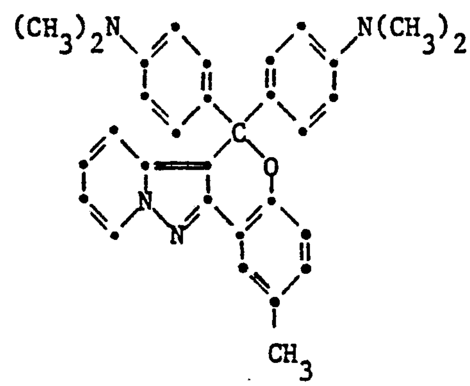

einen Schmelzpunkt von 230 bis 232° C

Auf Säureton entwickelt dieser Farbbildner eine intensive blaue farbe.

Das in Beispiel 3 als Ausgangsprodukt verwendete 2-(2-Hydroxy-5-methyl-phenyl)-3-azaindolizin wird folgendermassen hergestellt:

Zu 100 ml einer 15%igen n-Butyllithium-hexanlösung und 35 ml absolutem Ether werden bei -20° C unter Rühren eine Lösung von 15,6 g α-Picolin in 50 ml absolutem Ether während 50 Minuten zutropfen gelassen. Alsdann wird die Reaktionsmischung 30 Minuten am Rückfluss erwärmt. das Heizbad entfernt und 14,9 g Methyl-2-methoxy-5-methylbenzoat in 40 ml absolutem Ether so zutropfen gelassen, dass weiterhin leichter Rückfluss stattfindet. Nach der Beendigung der Benzoatzugabe wird die Reaktionsmischung 30 Minuten erwärmt, mit 40 ml Wasser versetzt und auf 100 g Eis/konz. Salzsäure (1:1) gegossen. Das Gemisch wird mit insgesamt 450 ml 6N-Salzsäure mehrfach extrahiert, worauf die vereinigten Extrakte mit 30%iger wässriger Natriumhydroxidlösung partiell neutralisiert und mit Natriumhydrogencarbonat auf pH 7 eingestellt werden. Die neutrale Lösung wird mit Ether ausgezogen, die Etherextrakte über wasserfreiem Natriumsulfat getrocknet und eingedampft. Der Rückstand

wird am Hochvakuum bei 143-147°C/ 11-12 Pa destilliert. Man erhält 13,1 g 2-(2-Methoxy-5-methylphenacyl)pyridin in Form eines gelben Oeles.

4,0 g dieser Pyridinverbindung in 30 ml Dichlormethan werden bei 0-3°C unter Rühren zu einer Lösung von Mesitylsulfonylhydroxylamin in 30 ml Dichlormethan gegeben und 1 Stunde bei 0°C und 15 Stunden bei Raumtemperatur weitergerührt. Hierauf wird das Reaktionsprodukt nach Entfernung von auskristallisierter Mesitylsulfonsäure mit Toluol/ Dichlormethan/Ethylacetat (6:2:1) chromatographiert. Nach dem Eindampfen bleiben 2,44 g (62% der Theorie) 2-(2-Methoxy-5-methyl-phenyl)-3-azaindolizin als Oel zurück, das am Hochvakuum bei 155°C/9-10 Pa destilliert wird.

600 mg reines 2-(2-Methoxy-5-methyl-phenyl)-3-azaindolizin werden bei 100°C 6-7 Stunden mit 5 g Trimethylsilyljodid gerührt und mit Wasser versetzt. Hierauf wird das Gemisch mehrmals mit Toluol extrahiert. Die vereinigten Toluolauszüge werden mit wässeriger Natriumhydrogensulfitlösung geschüttelt, über wasserfreiem Natriumsulfat getrocknet, eingedampft und mit Toluol chromatographiert. Man erhält 488 mg (86% der Theorie) reines 2-(2-Hydroxy-5-methyl-phenyl)-3-azaindolizin der Formel

(iii) 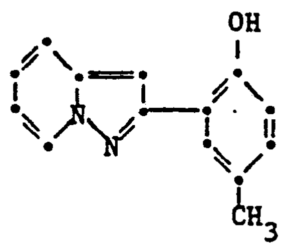

Nach Umkristallisation aus Petrolether schmilzt diese Verbindung bei 108°C.

**Beispiel 4: Herstellung eines druckempfindlichen Kopierpapiers**

Eine Lösung von 3 g der Chromeno-3-azaindolizin-Verbindung der Formel (13) (Beispiel 3) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive blaue Kopie, die ausgezeichnet lichtecht ist.

Entsprechende intensive und lichtechte blau-Kopien werden auch bei Verwendung der anderen in den Herstellungsbeispielen 1 und 2 angegebenen Farbbildner der Formeln (11) und (12) erzielt.

**Beispiel 5:** 1 g der Chromeno-1-azaindolizin-Verbindung der Formel (11) wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte blaue Farbe.

**Beispiel 6: Herstellung eines wärmeempfindlichen Aufzeichnungsmaterials**

In einer Kugelmühle werden 32 g 4,4'-Isopropyliden-diphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g der Chromeno-3-azaindolizinverbindung der Formel (13) (Beispiel 3), 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 g/m² auf ein Papier gestrichen. Durch Berührung des Papiers mit einem erhitzten Kugelschreiber wird eine intensive blaue Farbe erhalten, die eine ausgezeichnete Licht- und Sublimierechtheit hat.

Intensive und lichtechte blaue Farben werden auch bei Verwendung der anderen Farbbildner der Formeln (11)

und (12) gemäss Beispielen 1 und 2 erhalten.

**Beispiel 6:** In einer Kugelmühle werden 2,7 g der Chromeno-1-azaindolizinverbindung der Formel (11) (Beispi-el 1), 24 g N-Phenyl-N'-(1-hydroxy-2,2,2-trichlorethyl)-harnstoff, 16 g Stearinsäureamid, 59 g eines zu 88 % hydrolysierten Polyvinylalkohols und 58 ml Wasser gemahlen bis die Teilchengrösse 2-5 µm beträgt. Diese Suspension wird bei einem Trockenauftragsgewicht von 5,5 g/m$^2$ auf ein Blatt Papier gestrichen. Durch Berührung des Papiers mit einem erhitzten Kugelschreiber wird eine intensive und lichtechte blaue Farbe erhalten.

## Patentansprüche

1. Chromenoazaindolizine, dadurch gekennzeichnet, dass sie den allgemeinen Formeln

(1a) 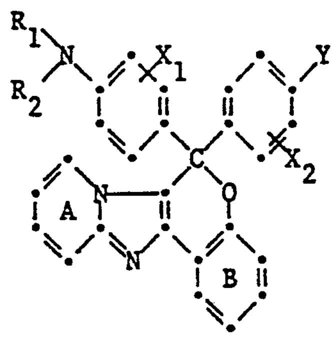 oder 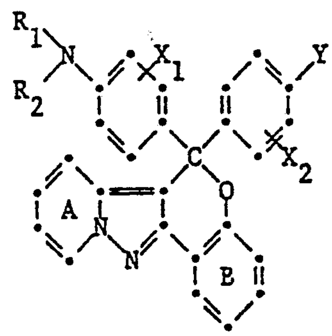 (1b)

entsprechen, worin
Y, $X_1$ und $X_2$, unabhängig voneinander, je Wasserstoff, Halogen, Niederalkyl, $C_2$-$C_5$-Alkanoylamino oder die Gruppen der Formeln (1c) -$OR_3$ oder

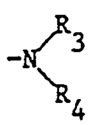 (1d),

$R_1$,$R_2$,$R_3$ und $R_4$ unabhängig voneinander Wasserstoff, unsubstituiertes oder durch Halogen, Hydroxyl, Cyano oder Niederalkoxy substituiertes Alkyl mit höchstens 12 Kohlenstoffatomen, Cycloalkyl, Phenyl, Benzyl oder durch Halogen, Nitro, Niederalkyl oder Niederalkoxy substituiertes Phenyl oder Benzyl oder

die Substituentenpaare ($R_1$ und $R_2$) und ($R_3$ und $R_4$), unabhängig voneinander, je zusammen mit dem sie verbindenden Stickstoffatom einen gesättigten, fünf- oder sechsgliedrigen heterocyclischen Rest bedeuten und

die Ringe A und B unabhängig voneinander unsubstituiert oder durch Halogen, Nitro, Niederalkyl, Niederalkoxy, Phenyl, Phenoxy oder durch eine gegebenenfalls durch Niederalkyl, Phenyl, Niederalkanoyl oder Benzyl mono- oder disubstituierte Aminogruppe substituiert sind, wobei

die Bezeichnung Nieder $C_1$-$C_5$; darstellt.

2. Chromenoazaindolizine gemäss Anspruch 1, dadurch gekennzeichnet, dass in den Formeln (Ia) und (Ib) Y eine Gruppe der Formel (1d) -$NR_3R_4$ bedeutet.

3. Chromenoazaindolizine gemäss Anspruch 1, dadurch gekennzeichnet, dass sie den Formeln

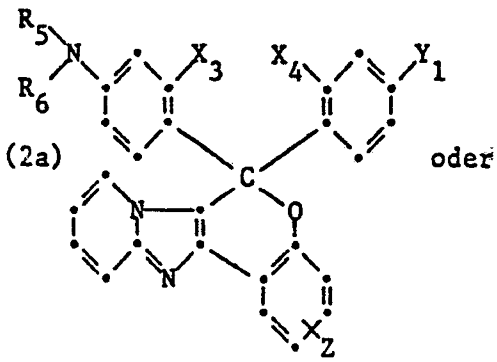

(2a) oder

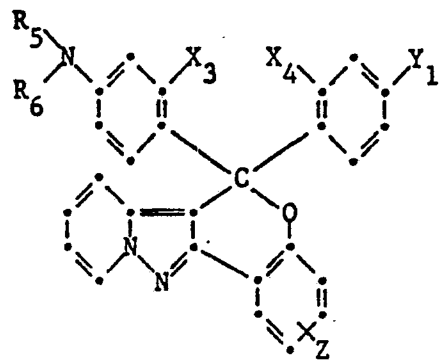

(2b)

entsprechen, worin

Y Wasserstoff, $-OR_7$ oder

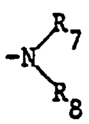

$X_3$ und $X_4$ unabhängig voneinander Wasserstoff, Halogen, Methyl, Methoxy, Ethoxy oder Acetylamino,

$R_5, R_6, R_7$

und $R_8$ unabhängig voneinander Niederalkyl, Cyanoethyl, Benzyl oder Phenyl

oder

die Substituentenpaare ($R_5$ und $R_6$) und ($R_7$ und $R_8$) unabhängigvoneinander Pyrrolidino, Piperidino oder Morpholino

und Z Wasserstoff, Halogen, Niederalkyl, Niederalkoxy, Phenyl oder eine gegebenenfalls durch Niederalkyl oder Benzyl oder Phenyl mono- oder disubstituierte Aminogruppe

bedeuten.

4. Chromenoazaindolizine gemäss Anspruch 3, dadurch gekennzeichnet, dass in den Formeln (2a) und (2b) $Y_1$ die Gruppe der Formel $-NR_7R_8$, $X_3$ und $X_4$ Wasserstoff und Z Wasserstoff, Halogen, Methyl oder Methoxy bedeuten.

5. Chromenoazaindolizine gemäss einem der Ansprüche 1 bis 3, dadurch gekennzeichnet, dass sie den Formeln (3a) und (3b)

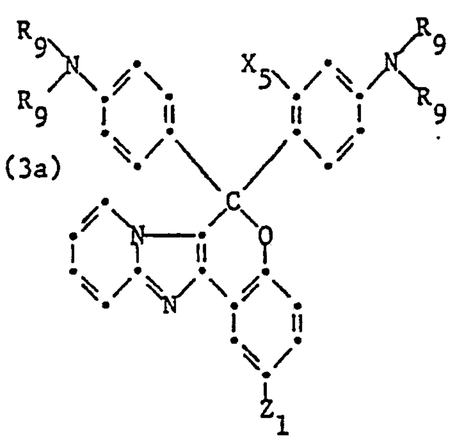

(3a) oder

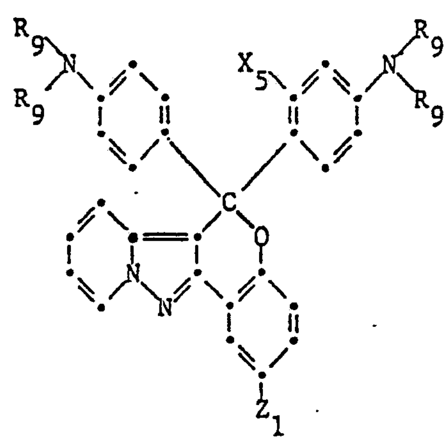

(3b)

entsprechen, worin

$R_9$ Niederalkyl, insbesondere Methyl oder Ethyl, $X_5$ Wasserstoff, Methoxy, oder Ethoxy und $Z_1$ Wasserstoff, Methyl, Methoxy oder Halogen bedeuten.

6. Chromenoazaindolizine gemäss Anspruch 5, dadurch gekennzeichnet, dass in den Formeln (3a) und (3b) $R_9$ Methyl oder Ethyl, $X_5$ Wasserstoff und $Z_1$ Wasserstoff, Chlor, Methyl oder Methoxy bedeuten.

7. Verfahren zur Herstellung von Chromeno-1-azaindolizinen der im Anspruch 1 angegebenen Formel (1a), dadurch gekennzeichnet, dass man eine 1-Azaindolizinverbindung der Formel

(4a) 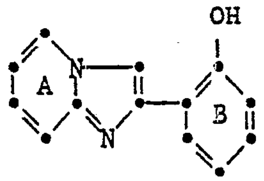

oder eine 1-Azaindoliziniumverbindung der Formel

(4b) 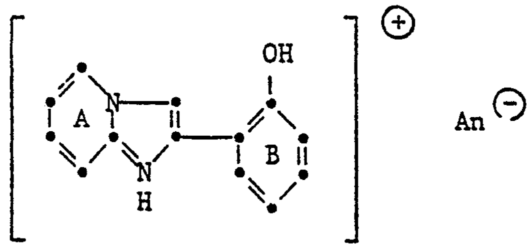

mit einer Carbinolverbindung der Formel

(5) 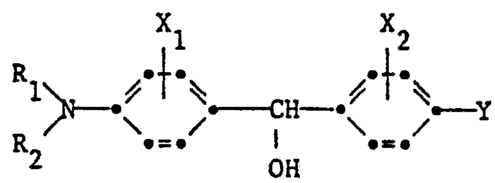

umsetzt und anschliessend das Reaktionsprodukt oxidiert, worin A, B, $R_1$, $R_2$, $X_1$, $X_2$ und Y die im Anspruch 1 angegebeneBedeutung haben und An⊖ das Anion einer organischen oder anorganischen Säure bedeutet.

8. Verfahren zur Herstellung von Chromeno-3-azaindolizinen der im Anspruch 1 angegebenen Formel (1b), dadurch gekennzeichnet, dass man eine 3-Azaindolizinverbindung der Formel

(6) 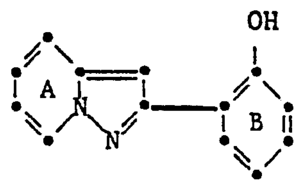

worin A und B die in Anspruch 1 angegebenen Bedeutungen haben, mit einer Carbinolverbindung der Formel

(5) 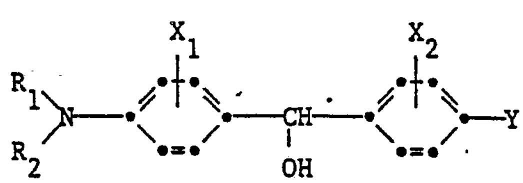

worin $R_1$, $R_2$, $X_1$, $X_2$ und Y die im Anspruch 1 angegebene Bedeutung haben, umsetzt und anschliessend das Reaktionsprodukt oxidiert.

9. Verwendung einer Chromenoazaindolizinverbindung der in einem der Ansprüche 1 bis 6 angegebenen Formeln als Farbbildner in einem druckempfindlichen oder wärmeempfindlichen Aufzeichnungsmaterial.

10. Druck- oder wärmeempfindliches Aufzeichnungsmaterial, dadurch gekennzeichnet, dass es in seinem Farbreaktantensystem als Farbbildner mindestens eine Chromenoazaindolizinverbindung der in einem der Ansprüche 1 bis 6 engegebenen Formeln enthält.

**Claims:**

1. A chromenoazaindolizine of the general formula

(1a) 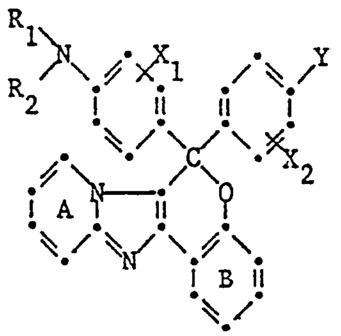 or 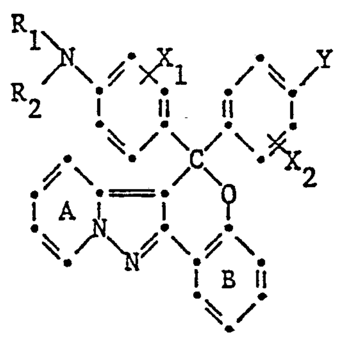 (1b)

wherein

each of Y, $X_1$ and $X_2$ independently of the other is hydrogen, halogen, lower alkyl, lower alkanoylamino or a group of the formula

(1c) $-OR_3$ or 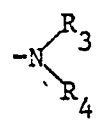 (1d),

each of $R_1$, $R_2$, $R_3$ and $R_4$ independently of the other is hydrogen, $C_1$-$C_{12}$alkyl which is unsubstituted or substituted by halogen, hydroxy, cyano or lower alkoxy, or is cycloalkyl, phenyl, benzyl, or phenyl or benzyl each substituted by halogen, nitro, lower alkyl or lower alkoxy, or

each pair of substituents ($R_1$ and $R_2$) and ($R_3$ and $R_4$) independently of the other, together with the nitrogen atom to which said pair is attached, is a 5- or 6-membered heterocyclic radical; and

each of the rings A and B independently of the other is unsubstituted or substituted by halogen, nitro, lower alkyl, lower alkoxy, phenyl, phenoxy, or by an amino group which is unsubstituted or mono- or disubstituted by lower alkyl, phenyl, lower alkanoyl or benzyl, the term ""lower" indicating that the radicals so qualified contain from 1 to 5 carbon atoms.

2. A chromenoazaindolizine according to claim 1, wherein Y is a group of the formula $-NR_3R_4$ (1d).

3. A chromenoazaindolizine according to claim 1 of the formula

(2a) 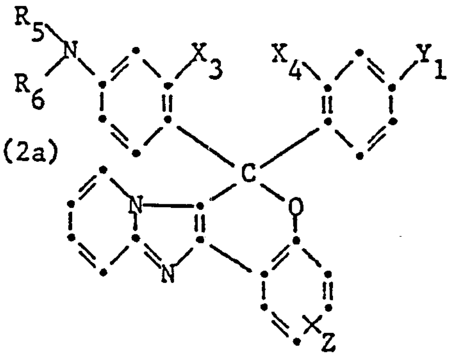 or 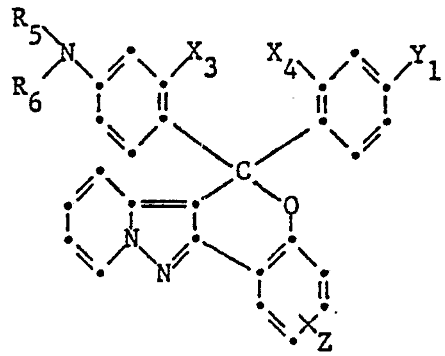 (2b) .

wherein Yl is hydrogen' $-OR_7$ or

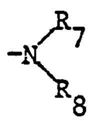 ,

each of $X_3$ and $X_4$ independently of the other is hydrogen, halogen, methyl, methoxy, ethoxy or acetylamino, each of $R_5$, $R_6$, $R_7$ and $R_8$ independently of the other is loweralkyl, cyanoethyl, benzyl or phenyl, or each pair of substituents ($R_5$ and $R_6$) and ($R_7$ and $R_8$) independently of the other is pyrrolidino, piperidino or morpholino, and

Z is hydrogen, halogen, lower alkyl, lower alkoxy, phenyl, or an amino group which is unsubstituted or mono- or disubstituted by lower alkyl, benzyl or phenyl.

4. A chromenoazaindolizine according to claim 3, wherein $Y_1$ is the group of the formula $-NR_7R_8$, $X_3$ and $X_4$ are hydrogen and Z is hydrogen, halogen, methyl or methoxy.

5. A chromenoazaindolizine according to any one of claims 1 to 3 of the formula

(3a) 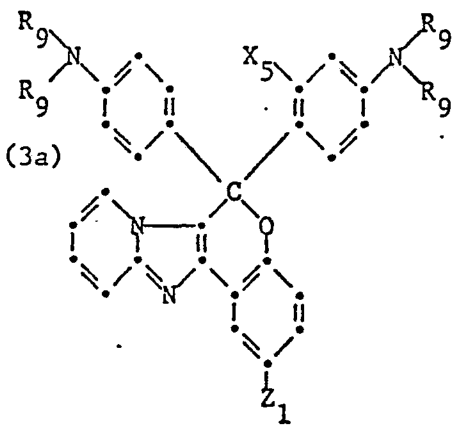 or 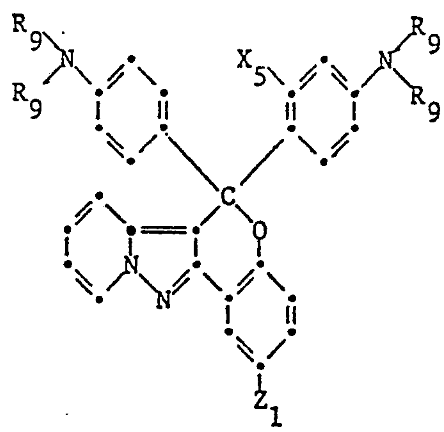 (3b)

wherein $R_9$ is lower alkyl preferably methyl or ethyl $X_5$ is hydrogen, methoxy or ethoxy, and $Z_1$ is hydrogen, methyl, methoxy or halogen.

6. A chromenoazaindolizine according to claim 5, wherien $R_9$ is methyl or ethyl X is hydrogen and Z is hydrogen, chlorine, methyl or methoxy.

7. A process for the preparation of a chromeno-1-azaindolizine of the formula (1a) as indicated in claim 1, which comprises reacting a 1-azaindolizine of the formula

(4a)

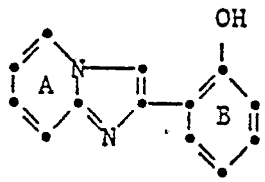

or a 1-azaindolizinium compound of the formula

(4b)

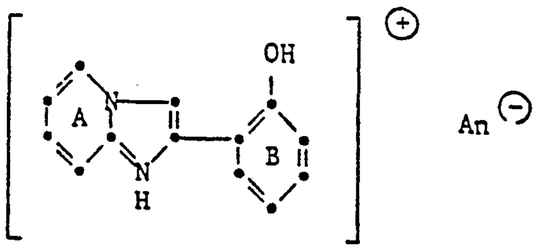

with a carbinol compound of the formula

(5)

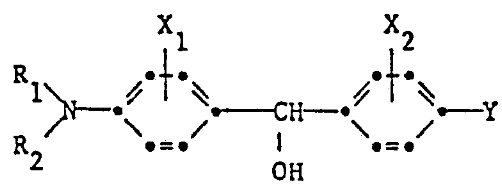

and subsequently oxidising the reaction product in which formulae above A, B, $R_1$, $R_2$, $X_1$, $X_2$ and Y are as defined in claim 1, and An ⊖ is the anion of an organic or inorganic acid.

8. A process for the preparation of a chromeno-3-azaindolizine of the formula (Ib) as indicated in claim 1, which comprises reacting a 3-azaindolizine of the formula

(5)

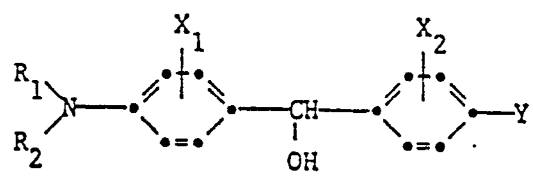

wherein A and B are as defined in claim 1, with carbinol compound of the formula

(6)

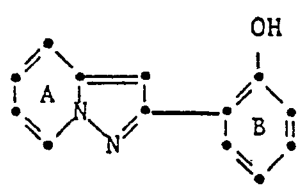

wherein $R_1$, $R_2$, $X_1$, $X_2$ and Y are as defined in claim 1, and subsequently oxidising the reaction product.

9. Use of a chromenoazaindolizine of the formula as indicated in any one of claims 1 to 6 as colour former in a pressure-sensitive or heat-sensitive recording material.

10. A pressure-sensitive or heat-sensitive recording material which contains in its colour reactant system, as colour former, at least one chromenoaza-indolizine of the formula as indicated in any one of claims 1 to 6.

**Revendications**

1. Chroménoazaindolizines, caractérisées par le fait qu'elles correspondent aux formules générales

(1a) 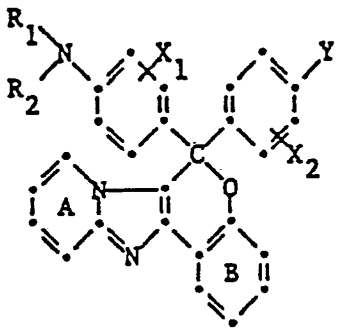 ou 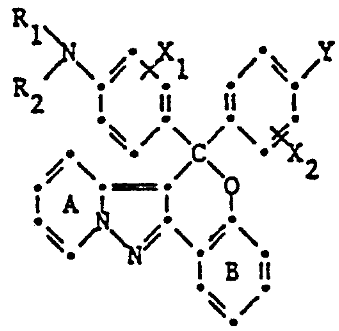 (1b)

dans lesquelles

Y, $X_1$ et $X_2$ désignent chacun, indépendamment les uns des autres, un radical hydrogène, halogène, alkyle inférieur, alcanoylamino inférieur ou les groupes de formules

(1c) $-OR_3$ ou 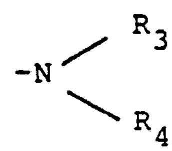 (1d),

$R_1$, $R_2$, $R_3$ et $R_4$ représentent indépendamment les uns des autres un radical hydrogène, alkyle à douze atomes de carbone au maximum non substitué ou substitué par un halogène, hydroxyle, cyano ou alcoxy inférieur, cycloalkyle, phényle, benzyle ou encore phényle ou benzyle substitué par un halogène, nitro, alkyle inférieur ou alcoxy inférieur ou

les paires de substituants ($R_1$ et $R_2$) et ($R_3$ et $R_4$), désignent chacune, indépendamment l'une de l'autre et conjointement avec l'atome d'azote auquel ces substituants sont reliés, un reste hétérocyclique saturé à cinq ou six chaînons,

et

les noyaux A et B sont indépendamment l'un de l'autre non substitués ou substitués par un halogène, nitro, alkyle inférieur, alcoxy inférieur, phényle ou phénoxy ou par un groupe amino éventuellement mono- ou disubstitué par un alkyle inférieur, phényle, alcanoyle inférieur ou benzyle,

le terme inférieur représentant un groupe en $C_1$-$C_5$.

2. Chroménoazaindolizines selon la revendication 1, caractérisées par le fait que, dans les formules (1a) et (1b), Y désigne un groupe de formules (Id) $-NR_3R_4$.

3. Chroménoazaindolizines selon la revendication 1, caractérisées par le fait qu'elles correspondent aux formules

(2a)
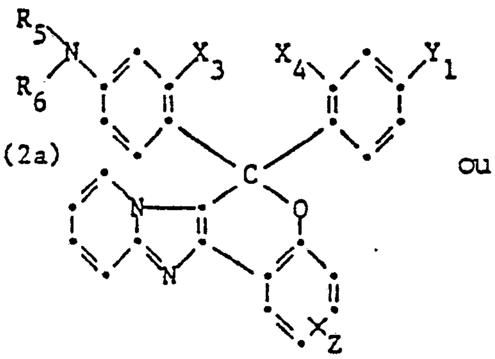
ou
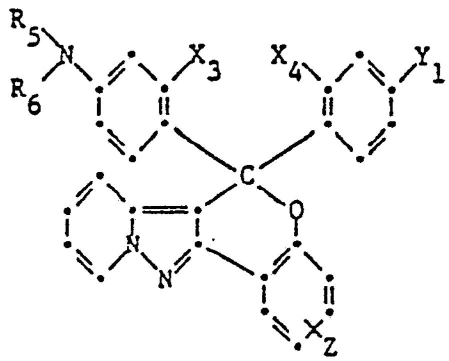
(2b).

dans lesquelles

$Y_1$ désigne l'hydrogène, $-OR_7$ ou

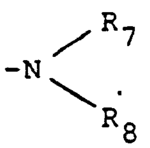

$X_3$ et $X_4$ désignent indépendamment l'un de l'autre l'hydrogène, un halogène, méthyle, méthoxy, éthoxy ou acétylamino,

$R_5$, $R_6$, $R_7$ et$R_8$ désignent indépendamment les uns des autres un alkyle inférieur, cyanoéthyle, benzyle ou phényle

ou

les paires de substituants ($R_5$ et $R_6$) et ($R_7$ et $R_8$) désignent indépendamment l'une de l'autre pyrrolidino, pipéridino ou morpholino

et Z désigne l'hydrogène, un halogène, alkyle inférieur, alcoxy inférieur, phényle ou un groupe amino éventuellement mono- ou disubstitué par des radicaux alkyle inférieur ou benzyle ou phényle.

4. Chroménoazaindolizines selon la revendication 3, caractérisées par le fait que dans les formules (2a) et (2b), $Y_1$ désigne le groupe de formule $-NR_7R_8$, $X_3$ et $X_4$ désignent chacun un hydrogène et Z désigne un hydrogène, un halogène, méthyle ou méthoxy.

5. Chroménoazaindolizines selon l'une quelconque des revendications 1 à 3, caractérisées par le fait qu'elles correspondent aux formules (3a) et (3b)

(3a)
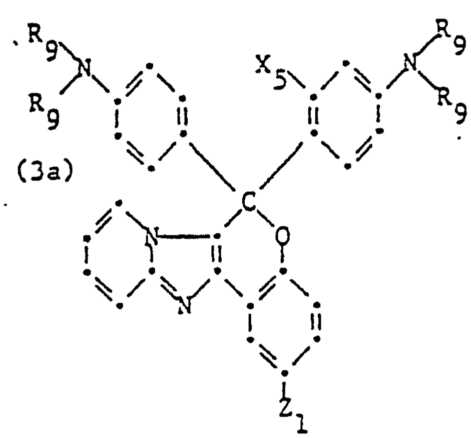
ou
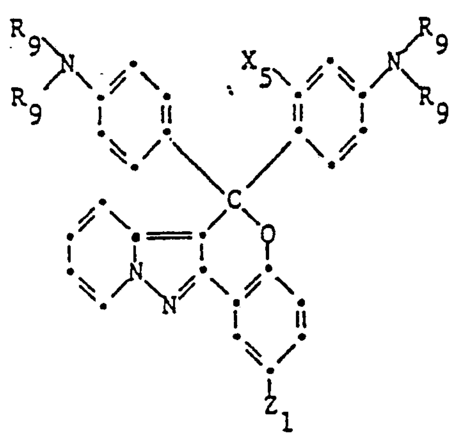
(3b)

dans lesquelles

$R_9$ désigne un alkyle inférieur, en particulier méthyle ou éthyle, $X_5$ désigne l'hydrogène, un méthoxy ou éthoxy et $Z_1$ désigne l'hydrogène, un méthyle, méthoxy ou halogène.

6. Chroménoazaindolizines selon la revendication 5, caractérisées par le fait que dans les formules (3a) et (3b), $R_9$ désigne un méthyle ou éthyle, $X_5$ désigne l'hydrogène et $Z_1$ désigne l'hydrogène, le chlore, un méthyle ou

méthoxy.

7. Procedé de preparation de chroméno-1-aza-indolizines de formule (Ia) indiquée dans la revendication 1, caractérisé par le fait que l'on fait réagir un composé 1-azaindolizine de formule

(4a)

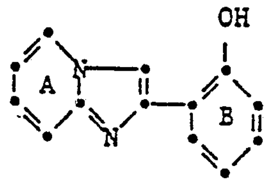

ou un composé 1-azaindolizinium de formule

(4b)

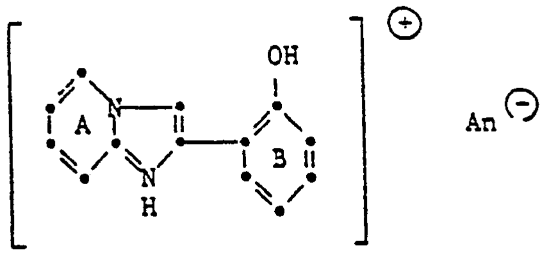

avec un composé carbinol de formule

(5)

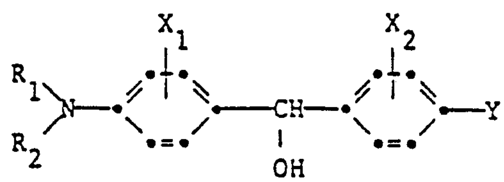

et on soumet consécutivement le produit de réaction à une oxydation, A, B, $R_1$, $R_2$, $X_1$, $X_2$ et Y étant dèfinis comme spécifié dans la revendication 1 et An désignant l'anion d'un acide organique ou inorganique.

8. Procédé de préparation de chroméno-3-azaindolizines de formule (Ib) indiquée dans la revendication 1, caractérisé par le fait que l'on fait réagir un composé 3-azaindolizine de formule

(6)

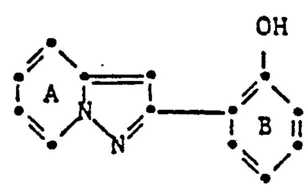

dans laquelle A et B sont définis comme spécifié dans la revendication 1, avec un composé carbinol de formule

(5) (5) 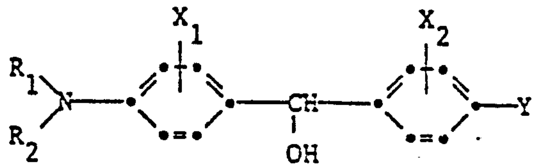

dans laquelle $R_1$, $R_2$, $X_1$, $X_2$ et Y sont définis comme spécifié dans la revendication 1 et on oxyde consécutivement le produit de réaction.

9. Application d'un composé chroménoazaindolizine correspondant aux formules indiquées dans l'une quelconque des revendications 1 à 6, en tant qu'agent chromogène dans un matériau de reproduction sensible à la pression ou sensible à la chaleur.

10. Matériau de reproduction sensible à la pression ou à la chaleur, caractérisé par le fait qu'il contient dans son système de réactifs colorés, en tant qu'agent chromogène au moins un composé chroménoazaindolizine des formules indiquées dans l'une quelconque des revendications 1 à 6.